# EUROPEAN PATENT APPLICATION

(11) **EP 2 876 164 A2**
(43) Date of publication of application: **27.05.2015**
(21) Application number: 14190080.3
(22) Date of filing: 23.10.2014
(51) Int. Cl.: C12N 15/87, A61K 51/08

(54) **Fusion peptide and use thereof for cell membrane penetrating**

(30) Priority: 29.10.2013 KR 20130129482
(71) Applicant: Samsung Electronics Co., Ltd, Gyeonggi-do 443-742 (KR)
(72) Inventor: Kim, Jung Min, Seoul (KR); Lee, Kyounghu, 445-787 Hwaseong-si (KR); Lee, Jae Il, Yongin-si (KR); Lee, Jungmin, Seoul (KR)
(74) Representative: Grünecker Patent- und Rechtsanwälte PartG mbB

(57) **Abstract**

Provided is a fusion peptide including a hydrophobic peptide and a basic peptide, a pharmaceutical composition including the fusion peptide, a cell membrane penetrating conjugate including the fusion peptide and a substance of interest, and a method for intracellular delivery of a substance of interest using the fusion peptide.

## Description

### BACKGROUND OF THE INVENTION

### 1. Field

Provided is a fusion peptide including a hydrophobic peptide and a basic peptide, a composition for cell membrane penetrating including the fusion peptide, a cell membrane penetrating conjugate including the fusion peptide and a substance of interest, and a method for intracellular delivery of a substance of interest using the fusion peptide.

### 2. Description of the Related Art

Various technologies for intracellular delivery of macromolecules such as proteins have been developed and spotlighted as a new therapeutic strategy. However, they have difficulties in accurately targeting to target cells or target organs. To solve such problems, there have been many studies on cell membrane penetration of proteins.

A protein transduction domain (PTD) was first suggested based on the finding that TAT protein from HIV-1 can be delivered inside a cell when it is added to a cell culture medium (Vives, E. et al., Tat peptide-mediated cellular delivery: back to basics, Advanced Drug Delivery Reviews 57, 2005). Thereafter, drosophila antennapedia (Antp) homeotic transcription factor (E. Bloch-Gallego et al., Antennapedia Homeobox Peptide Enhances Growth and Branching of Embryonic Chicken Motoneurons In Vitro, J.cell.Biol (1993)) and herpes simplex virus -1 DNA binding protein VP22 (Robert P. Bennett et al., Protein delivery using VP22, Nature Biotechnology (2002)) were also reported to be capable of penetrating a cell membrane, thereby being introduced into a cell.

Based on the fact that a fusion protein wherein PTDs are linked to other peptides or proteins can be delivered into a cell, various attempts have been made to transfer drugs, peptides, proteins, and the like, for therapeutic purpose into a cell using the PTDs.

### BRIEF SUMMARY OF THE INVENTION

An embodiment provides a fusion peptide including a hydrophobic peptide and a basic peptide.

Another embodiment provides a cell membrane penetrating conjugate including the fusion peptide and a substance of interest, such as a bioactive substance or contrast agent.

Additional embodiments provide a method for intracellular delivery of substance of interest using the fusion peptide, methods of preparing the fusion protein and conjugate, and related methods and compositions.

### BRIEF DESCRIPTION OF THE DRAWINGS

FIG. 1A and 1B provide fluorescent images showing cell membrane penetrability of MTS-BAA conjugate and a comparative conjugate for MCF-7 cell line.
FIG. 2 provides graphs showing the quantified fluorescent intensities of the fluorescent images shown in FIG. 1.
FIG. 3A and 3B provide fluorescent images showing cell membrane penetrability of MTS-BAA conjugate and a comparative conjugate for MDA-MB-231 cell line.
FIG. 4 is a graph showing the quantified fluorescent intensities of the fluorescent images shown in FIG. 3.
FIG. 5 provides fluorescent images showing cell uptake patterns of MTS-BAA conjugate in living MCF-7 cells, which indicates that the MTS-BAA conjugate are present in the cells in both the vesicle form and cytosol form. The upper section shows images of FITC labeled peptides, and lower section shows merged images.
FIG. 6A provides fluorescent images showing cell uptake of MTS-BAA conjugates in BT20 cells.
FIG. 6B provides fluorescent images showing cell uptake of MTS-BAA conjugates in MDA-MB-157 cells.
FIG. 6C provides fluorescent images showing cell uptake of MTS-BAA conjugates in HCC1806 cells.
FIG. 6D provides fluorescent images showing cell uptake of MTS-BAA conjugates in MDA-MB-231 cells.
FIG. 6E provides fluorescent images showing cell uptake of MTS-BAA conjugates in MCF7 cells.
FIG. 7 is a graph showing the quantified fluorescent intensities of the fluorescent images shown in FIGS. 6A-6E.
FIG. 8A and 8B provide fluorescent images showing cell uptake of various conjugates prepared in Example 5.1.
FIG. 9 is a graph showing the quantified fluorescent intensities of the fluorescent images shown in FIG. 8.
FIGS. 10A and 10B contain fluorescent images illustrating cell uptake of MTS-BAA conjugates observed in MCF7 cells in real time.
FIG. 10C contains images of electronic microscopy of MCF7 cells.
FIG. 10D is a graph showing the quantified fluorescent intensities of the fluorescent images of FIGS. 10A and 10B.
FIG. 11 is a graph showing relative viabilities of cells treated with an anticancer protein conjugate wherein the anticancer protein is conjugated to either a MTS-BAA fusion peptide or a TAT peptide.

### DETAILED DESCRIPTION OF THE INVENTION

Provided herein is a fusion peptide having a function as a cell membrane penetrating domain, a composition for cell membrane penetrating including the fusion peptide as an active ingredient, a cell membrane penetrating conjugate including the fusion peptide and a substance of interest, a method of preparing the cell membrane penetrating conjugate including conjugating the fusion peptide and a substance of interest, and a method for intracellular delivery of a substance of interest including administering the cell membrane penetrating conjugate to a subject, thereby delivering the substance of interest into a cell.

In some embodiments, the fusion peptide having a function as a cell membrane penetrating domain exhibits greater efficacy of cell membrane penetration than TAT-derived peptides, and, thus, it is expected to be useful not only in various intracellular studies but also in the treatment of diseases in need of intracellular delivery of drugs to a high efficiency. In such aspect, the fusion peptide having a function as a cell membrane penetrating domain and the cell membrane penetrating conjugate wherein a substance of interest is fused with the fusion peptide may be used as an effective drug delivery system.

An embodiment provides a fusion peptide including or consisting essentially of a hydrophobic peptide and a basic peptide.

The hydrophobic peptide may include a total of about 5 to about 100 amino acids, about 5 to about 50 amino acids, about 5 to about 40 amino acids, or about 6 to about 30 amino acids, and include hydrophobic amino acids at a ratio of about 60% or more, about 70% or more, about 80% or more, or about 90% or more, for example, about 60 to about 100%, about 70 to about 100%, about 80 to about 100%, or about 90 to about 100%, based on the number of the total amino acids in the hydrophobic peptide. The hydrophobic amino acid may refer to a non-charged, nonpolar, and/or neutral side chain-containing amino acid. The hydrophobic amino acid may be at least one selected from the group consisting of glycine, alanine, valine, leucine, isoleucine, methionine, proline, tryptophan, phenylalanine, and the like. That is, each hydrophobic amino acid included in the hydrophobic peptide may be independently selected from the group consisting of glycine, alanine, valine, leucine, isoleucine, methionine, proline, tryptophan, phenylalanine, and the like. The hydrophobic peptide may include one kind or two or more different kinds of hydrophobic amino acids selected from the hydrophobic amino acid group described above. When the hydrophobic peptide includes one kind of hydrophobic amino acid, the hydrophobic amino acid may be included once or repeatedly. In one embodiment, the hydrophobic peptide does not include a basic amino acid.

In a particular embodiment, the hydrophobic peptide may be at least one selected from the group consisting of a membrane-translocation sequence (MTS; for example, AAVALLPAVLLALLAP (SEQ ID NO: 1)), a peptide fragment of the MTS (for example, a peptide fragment including 7 to 16 consecutive amino acids within the amino acid sequence of SEQ ID NO: 1; e.g., AAVALLP (SEQ ID NO: 4), AVLLALLAP (SEQ ID NO: 5), etc.), a peptide including the amino acid sequence of AVLLALLAA (tMTS; SEQ ID NO: 17), a peptide including the amino acid sequence of AAVALLPAVLLALLAA (SEQ ID NO: 26), and the like.

The basic peptide may comprise, consisting essentially of or consisting of 1 to 6 basic amino acids (e.g., 2 to 6 basic amino acids). If the basic peptide includes 7 or more amino acids, the basic peptide acts as a cell penetrating peptide in itself, thereby transferring a protein to an endosome, leading to degradation of the protein. Therefore, it may be advantageous that the basic peptide includes 6 or fewer amino acids. The basic amino acid may be at least one selected from the group consisting of lysine, arginine, histidine, and the like, and for example, at least one selected from the group consisting of lysine and arginine. When the basic peptide includes two or more basic amino acids, each of the basic amino acids may be independently selected from the group consisting of lysine, arginine, histidine, and the like, and for example, selected from the group consisting of lysine and arginine. When the basic peptide includes one kind of basic amino acid, the basic amino acid may be included once or repeatedly.

In a particular embodiment, the basic peptide may include lysine (K), arginine (R), or a combination thereof, with 1 to 6 amino acid length. For example, the basic peptide may be at least one selected from the group consisting of KKKRK (SEQ ID NO: 2), KKKR (SEQ ID NO: 6), RKRK (SEQ ID NO: 7), RKRKRK (SEQ ID NO: 8), KKKKK (SEQ ID NO: 9), KKKKKR (SEQ ID NO: 10), KKKRKR (SEQ ID NO: 11), R5 (RRRRR; SEQ ID NO: 12), R6 (RRRRRR; SEQ ID NO: 13), or a combination thereof, and the like, but not be limited thereto. Some of the basic peptides have been known to have a nuclear membrane penetrating activity; however, none of them has been known to have a cell membrane penetrating activity. In the present disclosure, the basic peptide is fused (or linked, e.g., covalently) with a hydrophobic peptide, to produce a fusion peptide, thereby considerably increasing the cell membrane penetrating effect of the hydrophobic peptide or the fusion peptide.

The basic peptide may be linked (e.g., covalently) to the N-terminus or the C-terminus of the hydrophobic peptide, or linked to both of the N-terminus and the C-terminus of the hydrophobic peptide (when two or more basic peptides are included). One particular embodiment, in order to more increase the cell membrane penetrability, the basic peptide may be linked to the N-terminus or the C-terminus, for example the C-terminus, of the hydrophobic peptide. The basic peptide may be linked to the N-terminus or the C-terminus, for example the C-terminus, of the hydrophobic peptide, in forward direction (i.e., the N-terminus of the basic amino acid is linked to the C-terminus of the hydrophobic peptide, or the C-terminus of the basic amino acid is linked to the N-terminus of the hydrophobic peptide), or in reverse direction (i.e., the C-terminus of the basic amino acid is linked to the C-terminus of the hydrophobic peptide, or the N-terminus of the basic amino acid is linked to the N-terminus of the hydrophobic peptide). In an embodiment, the basic peptide may be linked to the N-terminus or the C-terminus, for example the C-terminus, of the hydrophobic peptide, in forward direction. When two or more basic peptides are respectively linked to both termini of the hydrophobic peptide, the basic peptides may be the same as or different from one another.

In a particular embodiment, the fusion peptide may include: a hydrophobic peptide and a basic peptide which is linked to the C-terminus of the hydrophobic peptide (that is, in the fusion peptide, a hydrophobic peptide is located at an N-terminal part and a basic peptide is located at a C-terminal part), or a hydrophobic peptide and a basic peptide which is linked to N-terminus of the hydrophobic peptide (that is, in the fusion peptide, a hydrophobic peptide is located at a C-terminal part and a basic peptide is located at an N-terminal part.

To further increase the cell membrane penetrability of the fusion peptide, the fusion peptide may include a hydrophobic peptide and a basic peptide which is linked to the C-terminus of the hydrophobic peptide.

As described above, the fusion peptide can have considerably increased cell membrane penetrability by including a hydrophobic peptide and basic peptide, for example, including a hydrophobic peptide at N-terminal part, and a basic peptide at C-terminal part. As illustrated in the examples, a representative cell membrane penetrating peptide, MTS (SEQ ID NO: 1), shows considerably increased cell membrane penetrating efficiency when it is fused with a basic peptide (SEQ ID NO: 2), compared with when it is used alone. Such results representatively indicate that the fusion peptide provided by the present disclosure can possess further increased cell membrane penetrating efficiency by including a hydrophobic peptide and a basic peptide. The fusion peptide can be usefully employed as a delivery system for delivering a substance of interest into a cell due to such increased cell membrane penetrating efficiency.

Therefore, another embodiment provides a pharmaceutical composition including the fusion peptide. In particular, a pharmaceutical composition for cell membrane penetrating including the fusion peptide is provided. The composition for cell membrane penetrating may be conjugated (e.g., covalently) with various substances of interest (for example, bioactive substances, contrast materials, etc.), thereby possessing a function to transport the substances to inside a cell.

The term "cell membrane penetrating" (cell membrane transferring) or "cell membrane penetrability" may refer to transporting a substance to the inside of a cell by passing through one or more of a cell membrane with a lipid bilayer ex vivo and/or in vivo, and in a particular case, the term excludes a transfer into a nucleus, unless otherwise stated.

The cell to be penetrated may be any cell having a cell membrane with a lipid bilayer. The cell may be any cell in need of intracellular delivery of a bioactive substance, such as therapeutic drugs and the like. For example, the cell may be a cell of lesion, such as cancer cells and the like, but not be limited thereto.

Another embodiment provides a cell membrane penetrating conjugate including the fusion peptide and a substance of interest.

The cell membrane penetrating conjugate may include at least one substance of interest at one or more positions selected from the group consisting of the N-terminus of the fusion peptide, the C-terminus of the fusion peptide, and a linking part between the hydrophobic peptide and basic peptide. To further increase the cell membrane penetrability of the cell membrane penetrating conjugate, the substance of interest may be conjugated at the C-terminus or the N-terminus of the fusion peptide, for example, at the C-terminus of the fusion peptide.

In particular, the cell membrane penetrating conjugate may include:
a hydrophobic peptide, a basic peptide which is linked to the C-terminus of the hydrophobic peptide, and a substance of interest which is linked to the C-terminus of the basic peptide,
a hydrophobic peptide at an N-terminal part of the conjugate, a basic peptide at a C-terminal part of the conjugate, and a substance of interest which is linked between the C-terminus of the hydrophobic peptide and the N-terminus of the basic peptide,
a hydrophobic peptide, a substance of interest which is linked to the N-terminus of the hydrophobic peptide, and a basic peptide which is linked to the C-terminus of the hydrophobic peptide,
a basic peptide, a hydrophobic peptide which is linked to the C-terminus of the basic peptide, and a substance of interest which is linked to the C-terminus of the hydrophobic peptide,
a basic peptide at an N-terminal part of the conjugate, a hydrophobic peptide at a C-terminal part of the conjugate, and a substance of interest which is linked between the C-terminus of the basic peptide and the N-terminus of the hydrophobic peptide, or
a basic peptide, a substance of interest which is linked to the N-terminus of the basic peptide, and a hydrophobic peptide which is linked to the C-terminus of the basic peptide.

In some embodiments, the hydrophobic peptide, basic peptide, and substance of interest may be linked directly to one another in the order described above.

The cell membrane penetrating conjugate may further include at least one basic peptide (hereinafter, "second basic peptide" or "nuclear membrane penetrating peptide" for distinguishing from the basic peptide ("first basic peptide") included in the fusion peptide) at an N-terminal part and/or a C-terminal part of the conjugate, or inside the conjugate, in addition to the fusion peptide and a substance of interest. The features of the basic peptide used as the nuclear membrane penetrating peptide are as described above description with respect to the basic peptide included in the fusion peptide. In a given fusion peptide or conjugate, the nuclear membrane penetrating peptide may be the same (e.g., has the same amino acid sequence) as the basic peptide included in the fusion peptide of the conjugate, or it may be different. The nuclear membrane penetrating peptide may be included at the N-terminus, the C-terminus, or both termini of the conjugate in which the fusion peptide and substance of interest are linked to each other, or between (at the junction or linking part of) the fusion peptide and the substance of interest. In a particular embodiment, the cell membrane penetrating conjugate may include a hydrophobic peptide, a basic peptide linked to the C-terminus of the hydrophobic peptide, a substance of interest linked to the C-terminus of the basic peptide, and nuclear membrane penetrating conjugate linked to the C-terminus of the substance of interest (in case the substance of interest is a peptide or a protein).

The hydrophobic peptide, the basic peptide, and/or the substance of interest included in the fusion peptide or conjugate may be linked (e.g., fused or conjugated) to each other directly (with no linker) or via a peptide linker. When they are linked to each other via a peptide linker, the peptide linker may be a peptide including about 1 to about 100 amino acids, about 2 to about 50 amino acids, about 1 to about 20 amino acids or about 2 to about 10 amino acids, wherein each of the amino acids may be independently selected from any amino acids with no limitation. The peptide linker may include at least one amino acid residue selected from the group consisting of Gly, Asn, Ser, and the like, and/or at least one neutral amino acid selected from the group consisting of Thr, Ala, and the like. Several appropriate amino acid sequences usefully employed as the peptide linker are well known to the relevant art.

The substance of interest may be any substance which is required to be delivered to inside a cell for various purpose such as treatment, diagnosis, and the like, and may be at least one selected from the group consisting of any bioactive substances and any contrast materials.

The bioactive substance may refer to any biocompatible substances capable of functioning and exhibiting advantageous effects in vivo or ex vivo. The bioactive substance may be at least one selected from the group consisting of various proteins, peptides, nucleic acids (e.g., DNA, RNA, siRNA, shRNA, microRNA, etc.), chemical drugs, and the like, and for example, the bioactive substance may be at least one selected from the group consisting of proteins and peptides.

A bioactive protein may be at least one selected from the group consisting of any proteins having a molecular weight of about 2 KDa to about 150 KDa, and for example, the protein may be at least one selected from the group consisting of antibodies (e.g., at least one selected from the group consisting of IgA, IgD, IgG (e.g., IgG1, IgG2, IgG3, or IgG4), IgE, IgM, and the like), antigen-binding fragments of the antibodies (e.g., at least one selected from the group consisting of scFv, scFvFc, (scFv)₂, Fab, Fab', F(ab')₂, and the like), hormones, hormone analogues, enzymes, tumor suppressors, signal transduction proteins, receptors, adhesion proteins, structural proteins, regulatory proteins, toxoproteins, cytokines, transcription factors, hemocoagulation factors, and the like.

A bioactive peptide may include at least 2 amino acids, for example, about 5 to about 100 amino acids, about 10 to about 50 amino acids, or about 15 to about 45 amino acids. The peptide may be at least one selected from the group consisting of (D)pMI-alpha (TNWYANLEKLLR; SEQ ID NO: 14), (D)pMI-beta (TAWYANFEKLLR; SEQ ID NO: 3), p53 fragment (SQETFSDLWKLLPEN; SEQ ID NO: 15), various growth factors, aptamers including about 10 to about 50 amino acids or about 15 to about 45 amino acids, and the like.

In a particular embodiment, the peptide or the protein may be at least one selected from the group consisting of p15, p16, p18, p53, p21, p25, p57, p16 variants (e.g., SEQ ID NO: 16, etc.), NIP71, neuroregulin 1, PTEN (phosphatase and tensin homolog) tumor suppressor, ARF tumor suppressor, APC, CD95, folliculin, MEN1, BRCA1, Von Hippel-Lindau tumor suppressor, RKIP, nm23, endostatin, insulin, IGF-1 (insulin-like growth factor 1), growth hormones, erythropoietin, G-CSFs (granulocyte-colony stimulating factors), GM-CSFs (granulocyte/macrophage-colony stimulating factors), interferon-alpha, interferon-beta, interferon-gamma, interleukin-1 alpha, interleukin-1 beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II(GHRH-II), gonadorelin, goserelin, histrelin, leuprorelin, lypressin, octreotide, oxytocin, pitressin, secretin, sincalide, terlipressin, thymopentin, thymosine α1, triptorelin, bivalirudin, carbetocin, cyclosporine, exedine, lanreotide, luteinizing hormone-releasing hormone (LHRH), nafarelin, parathyroid hormone (PTH), pramlintide, T-20 (enfuvirtide), thymalfasin, ziconotide, (D)pMI-alpha (TNWYANLEKLLR; SEQ ID NO: 14), (D)pMI-beta (TAWYANFEKLLR; SEQ ID NO: 3), p53 fragment (SQETFSDLWKLLPEN; SEQ ID NO: 15), and the like.

The nucleic acids may be single-stranded or double-stranded one in lengths of 1 to about 100 bp, about 2 to about 70 bp, about 5 to about 50 bp, or about 10 to about 40 bp, and may be at least one selected from the group consisting of DNAs, RNAs, small interfering RNAs (siRNAs), small hairpin RNAs (shRNAs), micro RNAs (miRNAs), and the like.

The chemical drug may be any compounds having a molecular weight of about 2 KDa to about 150 KDa, capable of being used for treating, alleviating, improving, diagnosing, and/or regulating various diseases. For example, the chemical drug may be at least one selected from the group consisting of various anti-cancer agents, anti-inflammatory agents, immune-regulatory agents, and the like, such as, nutilin 3a, PD0332991, and the like.

The contrast material may be any compounds having a molecular weight of about 2 KDa to about 150 KDa, capable of being used for the visualization of cells. For example, the contrast material may be at least one selected from the group consisting of endosome markers (e.g., anti-EEA1 (early endosome antigen) antibody, mannose-6-phosphate receptor, anti-Rab4 antibody, anti-Rab5 antibody, anti-LAMP-1 (lysosome-associated membrane protein-1) antibody, etc.), golgi markers (e.g., anti-58K golgi protein antibody, anti-mannosidase II antibody, etc.), Cre recombinase (e.g., DQ023272.1, etc.), integrase (e.g., phiC31, X59938, etc.), and the like. The markers and/or enzymes may be in a form of conjugate which is conjugated with a coloring material, a fluorescent material, or a luminous material.

When a bioactive substance is included in a cell membrane penetrating conjugate, the cell membrane penetrating conjugate may be usefully applied for cell membrane penetration or intracellular delivery of the bioactive substance.

Therefore, one embodiment provides a pharmaceutical composition for cell membrane penetration or intracellular delivery of a bioactive substance, wherein the composition includes a fusion peptide and a bioactive substance (e.g., a conjugate), and a pharmaceutically acceptable carrier.

Another embodiment provides a method of cell membrane penetration or intracellular delivery of a substance of interest (for example, a bioactive substance), using a fusion peptide.

In particular, the method of cell membrane penetration or intracellular delivery of a substance of interest may include administering the fusion peptide and a bioactive substance, or the cell membrane penetrating conjugate, to a subject. The method may further include a step of identifying a subject in need of delivery (e.g., intracellular delivery) of the substance of interest included in the cell membrane penetrating conjugate, prior to the administration step.

The subject may be any animal selected from mammals such as primates including human, monkeys, etc., rodents including rats, mice, etc., and the like, a cell, a tissue, or body fluid (e.g., serum) derived (isolated) from the animal; or a culture thereof. The subject may be an animal, or a cell, a tissue, or body fluid derived (isolated) from the animal (living body), which is in need of delivery (e.g., intracellular delivery) of the substance of interest included in the cell membrane penetrating conjugate.

The fusion peptide or the cell membrane penetrating conjugate may be administered to a subject in need of administration of the substance of interest, via oral or parenteral route, or administered by being contacted with a cell, tissue, or body fluid isolated from the subject (living body).

The pharmaceutical composition may further include or the conjugate or the fusion peptide may be administered with a pharmaceutically acceptable carrier. The pharmaceutically acceptable carrier may be any one that is commonly used in formulation of drugs, and may be, but not limited to, at least one selected from the group consisting of lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, mineral oil, and the like. The pharmaceutically composition may further include at least one selected from the group consisting of a diluent, an excipient, a lubricant, a wetting agent, a sweetener, a flavor enhancer, an emulsifying agent, a suspension agent, a preservative, and the like.

The fusion peptide or the cell membrane penetrating conjugate or the composition may be administered via oral or parenteral route. Parenteral administration may be performed by intravenous injection, subcutaneous injection, muscular injection, intraperitoneal injection, endothelial administration, local administration, intranasal administration, intrapulmonary administration, and/or rectal administration. Since oral administration leads to digestion of proteins or peptides, an active ingredient in the compositions for oral administration must be coated or formulated to prevent digestion in stomach.

In addition, the fusion peptide or the cell membrane penetrating conjugate may be in a form of solution in oil or an aqueous medium, suspension, syrup, or emulsifying solution form, or may be formulated into a form of an extract, powders, granules, a tablet or a capsule. The cell membrane penetrating conjugate may further include a dispersing agent and/or a stabilizing agent for its formulation.

When the cell membrane penetrating conjugate include a contrast material as a substance of interest, the conjugate can be used for visualization of a cell or a cell component (e.g., cytoplasm).

Therefore, one embodiment provides a composition for visualization of a cell (e.g., cytoplasm), which includes a fusion peptide and a contrast material. Another embodiment provides a method of visualization of a cell including administering a cell membrane penetrating conjugate including a fusion peptide and a contrast material to a subject. The method may further comprise visualizing the cell (e.g., the contrast material in the cell) by any suitable technique.

The subject may be any animal selected from mammals such as primates including human, monkeys, etc., rodents including rats, mice, etc., and the like; a cell, a tissue, or body fluid (e.g., serum) derived (isolated) from the animal (living body); or a culture thereof. The subject may be in need of visualization of a cell. The cell membrane penetrating conjugate including a contrast material may be administered to a subject in need of visualization of a cell, via oral or parenteral route, or administered by being contacted with a cell, tissue, or body fluid isolated from the subject (living body).

The cell to be visualized by the composition or via a method for visualization of a cell may be a normal cell or a cell from lesion, for example, the cell may be a normal cell or a cancer cell.

Another embodiment provides a method of preparing a fusion peptide having cell membrane penetrability or a method of improving cell membrane penetrability of a hydrophobic peptide, including covalently linking (e.g., fusing or conjugating) a basic peptide to the N-terminus, the C-terminus, or both termini (in case at least two basic peptides are used) of a hydrophobic peptide. The linking step may be performed ex vivo. The linking also may be performed by providing a nucleic acid encoding the fusion peptide, and expressing the nucleic acid in a cell.

Another embodiment provides a method of improving cell membrane penetrability of a substance of interest, including covalently linking (e.g., fusing or conjugating) a hydrophobic peptide, a basic peptide, and a substance of interest to one another, or linking a substance of interest to the fusion peptide. The linking step may be performed ex vivo. When the substance of interest is a peptide or protein, the linking also may be performed by providing a nucleic acid encoding a fusion protein comprising the hydrophobic peptide, basic peptide, and substance of interest, and expressing the nucleic acid in a cell.

In a particular embodiment, the method of improving cell membrane penetrability of a substance of interest may include:
providing a fusion peptide including a hydrophobic peptide and a basic peptide linked to the N-terminus, the C-terminus, or both termini (in case at least two basic peptide are used) of the hydrophobic peptide, and
linking a substance of interest to at least one selected from the N-terminus and the C-terminus of the fusion peptide and a linking part between a hydrophobic peptide and a basic peptide in the fusion peptide, to produce a conjugate. To increase cell membrane penetrability, the substance of interest may be linked to the N-terminus or the C-terminus of the fusion peptide, for example, to the C-terminus of the fusion peptide.

The step of providing a fusion peptide including a hydrophobic peptide and a basic peptide which are linked to each other may be performed by linking a basic peptide to the N-terminus, the C-terminus, or both termini (in case at least two basic peptides are used) of the fusion peptide, for example, to the C-terminus of the fusion peptide a hydrophobic peptide, or acquiring the fusion peptide including a hydrophobic peptide and a basic peptide which are linked to each other. The step of linking a substance of interest to a fusion peptide may be performed simultaneously with or after the step of providing a fusion peptide.

The method of improving cell membrane penetrability of a substance of interest may further include a step of linking at least one basic peptide (hereinafter, "nuclear membrane penetrating peptide" for distinguishing from the basic peptide included in the fusion peptide) to the N-terminus and/or the C-terminus of the produced conjugate, for example, to the C-terminus of the produced conjugate. The step of linking a nuclear membrane penetrating peptide may be performed ex vivo.

The hydrophobic peptide, the basic peptide, the nuclear membrane penetrating peptide, and the substance of interest may refer to the description herein.

The present disclosure suggests a cell membrane penetrating domain having more excellent cell membrane penetrability than pre-existing cell membrane penetrating peptides, and an intracellular delivery system including a cargo to be transferred inside a cell, thereby carrying out an efficient intracellular delivery.

Hereafter, the present disclosure will be described in detail by examples.

The following examples are intended merely to illustrate the invention and should in no way be interpreted as limiting the invention.

### EXAMPLES

### Example 1. Preparation of a Cell Membrane Penetrating Conjugate

### 1.1. Preparation of Conjugate, (L)MTS-(L)BAA-(D)pMI-beta

A conjugate having the following amino acid sequence was prepared by ANYGEN Co. Ltd. and PEPTRON Inc.:
FITC-AAVALLPAVLLALLAP-KKKRK-TAWYANFEKLLR-NH₂ [FITC-(L)MTS-(L)BAA-(D)pMI-b] [FITC-(SEQ ID NO: 1)-(SEQ ID NO: 2)-(SEQ ID NO: 3)-NH₂] (wherein BAA refers to a basic peptide, KKKRK; and FITC is fluorescein isothiocyanate).

The synthesized conjugate was named as "MTS-BAA".

### 1.2. Preparation of Comparative Conjugates

Conjugates as shown in Table 1 were prepared by ANYGEN Co. Ltd. and PEPTRON Inc.:

**[Table 1]**

| Name | Molecule | Sequence (FITC-X-NH2 or Ac-X-K-FITC) |
|---|---|---|
| MTS | FITC-(L)MTS-(D)pMI-b | 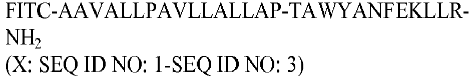 |
| TAT | FITC-(L)TAT-(D)pMI-b | FITC-RKKRRQRRR-TAWYANFFKLLR-NH₂ |
| | | (X: SEQ ID NO: 20-SEQ ID NO: 3) |
| MTD103 | FITC-(L)MTD 103-(D)pMI-b | FITC-LALPVLLLA-TAWYANFEKLLR-NH₂ |
| | | (X: SEQ ID NO: 21-SEQ ID NO: 3) |
| TP10 | FITC-(L)TP10-(D)pMI-b | 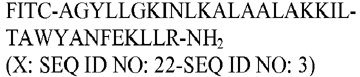 |
| Penetratin | FITC-(L)Penetratin-(D)pMI-b | 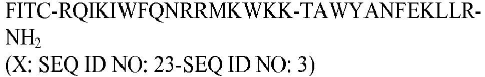 |
| R9 | FITC-(L)R9-(D)pMI-b | 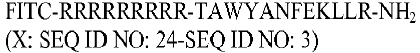 |
| MAP | FITC-(L)MAP-(D)pMI-b | 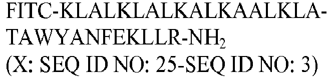 |

### 1.3. Preparation of Various Cell Membrane Penetrating Conjugates

To reveal the role of proline in a hydrophobic peptide of SEQ ID NO: 1, various conjugate of "MTS-BAA" family as shown in Table 2 were prepared by ANYGEN Co. Ltd. and PEPTRON Inc.

**[Table 2]**

| | Molecule | Sequence (FITC-X-NH2 or Ac-X-K-FITC) |
|---|---|---|
| 21 | FITC-(L)MTS-BAA-(D)pMI-b | FITC-AAVALLPAVLLALLAP-KKKRK- TAWYANFEKLLR-NH2 (X: SEQ ID NO: 1-SEQ ID NO: 2-SEQ ID NO: 3) |
| 25 | FITC-(L)tMTS-BAA-(D)pMI-b | FITC-AVLLALLAP-KKKRK-TAWYANFEKLLR-NH₂ |
| | | (X: SEQ ID NO: 5-SEQ ID NO: 2-SEQ ID NO: 3) |
| 26 | FITC-(L)MTS-BAA-(D)pMI- b-BAA | FITC- AAVALLPAVLLALLAP -TAWYANFEKLLR- KKKRK-NH2 (X: SEQ ID NO: 1-SEQ ID NO:3 -SEQ ID NO: 2) |
| 27 | FITC-(L)MTS-BAA'-(D)pMI-b | FITC- AAVALLPAVLLALLAP-RRRRR- |
| | | TAWYANFEKLLR-NH2 (X: SEQ ID NO: 1-SEQ ID NO: 12--SEQ ID NO: 3) |
| 29 | FITC-BAA-(L)MTS-(D)pMI-b | FITC- KKKRK-AAVALLPAVLLALLAP - TAWYANFEKLLR-NH2 (X: SEQ ID NO: 2-SEQ ID NO: 1-SEQ ID NO: 3) |
| 30 | FITC-(L)tMTS-BAA-(D)pMI-b-cont | FITC-AVLLALLAA -KKKRK-TAWYANFEKLLR-NH₂ |
| | | (X: SEQ ID NO: 17-SEQ ID NO: 2-SEQ ID NO: 3) |
| 31 | FITC-(L)MTS-BAA-(D)pMI-b-BAA-cont | FITC- AAVALLPAVLLALLAA-KKKRK - TAWYANFEKLLR-KKKRK-NH2(X: SEQ ID NO: 26- SEQ ID NO: 2-SEQ ID NO: 3-SEQ ID NO: 2) |
| 32 | FITC-(L)MTS-BAA-(D)pMI-b-cont | FITC- AAVALLPAVLLALLAA-RRRRR - TAWYANFEKLLR-NH2 (X: SEQ ID NO: 26-SEQ ID NO: 12-SEQ ID NO: 3) |
| 33 | FITC-BAA-(L)MTS-(D)pMI-b-cont | FITC- KKKRK-PAAVALLPAVLLALLAP - TAWYANFEKLLR-NH2 (X: SEQ ID NO:2-SEQ ID NO: 1-SEQ ID NO: 3) |

| | | |
|---|---|---|
| (BAA: basic peptide, KKKRK; BAA': basic peptide, R5 (i.e. RRRRR)) | | |

### Example 2. Test of Cell Membrane Penetrability of the Cell Membrane Penetrating Conjugates

The conjugates prepared in Examples 1.1 to 1.3 were subjected to a cell membrane penetrability teat for MCF-7 cells and MDA-MB-231 cells.

The cell membrane penetrability teat was conducted as follows.

To compare intracellular uptake levels of the prepared cell membrane penetrating conjugates, MCF-7 cells (10,000 cells/well; ATCC, HTB-22) were cultured in 8 well imaging chamber (Ibidi) for 20 hours, and then, were treated with each of the cell membrane penetrating conjugates at a concentration of 1 µM (micromole) or 5 µM for 1 or 3 hours. Thereafter, the medium was exchanged with 10% (v/v) FBS-containing RPMI1640 medium (Invitrogen).

The obtained cultured cells were subjected to imaging and image analysis as follows. For imaging, a confocal microscope (LSM710, Carl Zeiss) and Live cell Chamber (LCI; Live cell instrument) were used. Four average imaging was conducted using an x63 objective lens (ZEISS Plan-Apochromat 63x/1.4 oli DIC) at a resolution of 1024x1024. Digital zoom (1- to 2.5-fold) and a range indicator were used for gain/offset control. The imaging was conducted with LCI setting under the conditions of 37°C and 5% CO₂, and maximum imaging tine were set as 2.5 hours. The obtained cell images were analyzed using Zen (Carl Zeiss) and image J (public), and plotted using Excel (Microsoft) and Sigmaplot. Image J was of a version equipped with 'UCSD Confocal Microscopy Plugins and MBF ImageJ for Microscopy Collection by Tony Collins'.

The intracellular uptake level was measured by determining cell areas in the obtained images and quantifying total intensity of each cell area. When performing time-lapse, colors of CPP-peptide images were converted into rainbow using Image J.

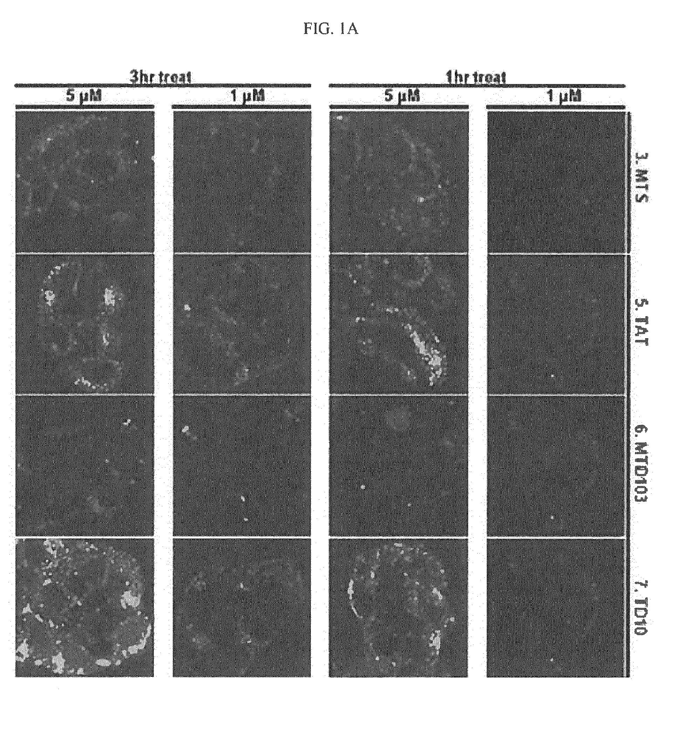

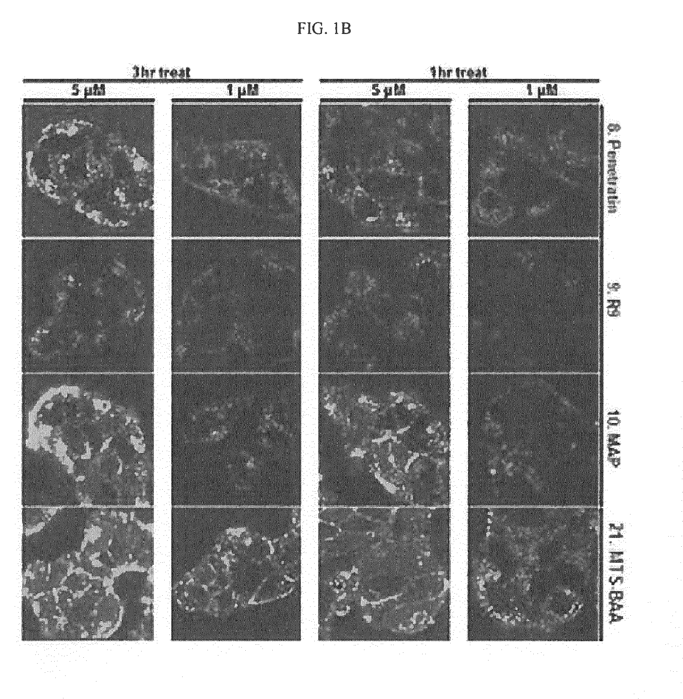

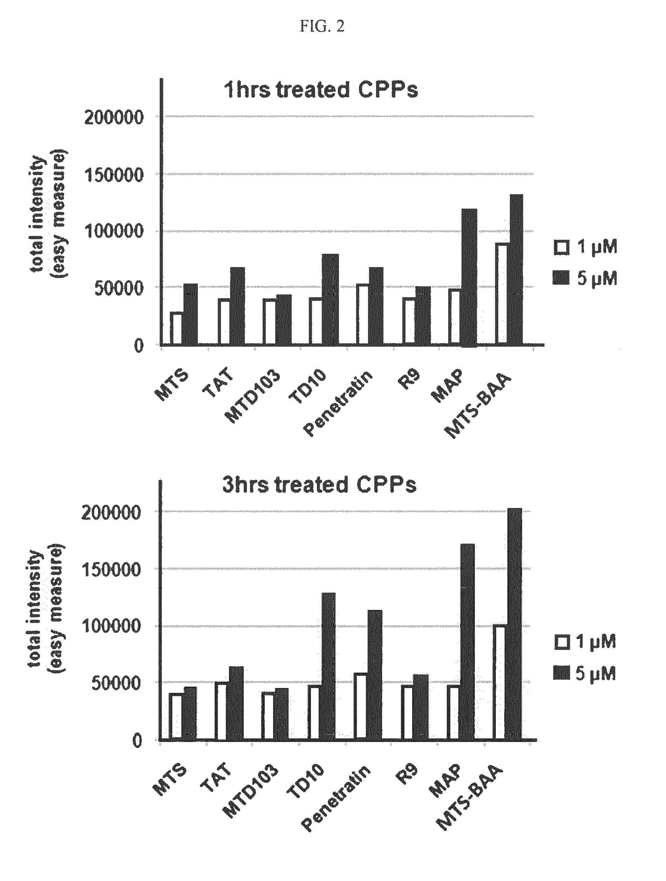

The obtained results are illustrated in FIGS. 1A, 1B, and 2. FIG. 2 is s graph illustrating numerical values obtained by quantifying fluorescent intensities shown in FIGS. 1A and 1B.

The cell membrane penetrability for MDA-MB-231 cells was examined as follows.

MDA-MB-231 cells (10,000 cells/well; ATCC, HTB-26) were cultured in 8 well imaging chamber (Ibidi) for 20 hours, and then, treated with each of the cell membrane penetrating conjugates at a concentration of 1 µM or 3 µM for 2 hours. Thereafter, the medium was exchanged with 10% (v/v) FBS containing RPMI1640 medium (Invitrogen). Imaging (total imaging magnification: (ocular lens X 10) X 630) was conducted, and then, the images obtained from the imaging was analyzed using image J and plotted. The imaging and image analysis were conducted referring to the method described above.

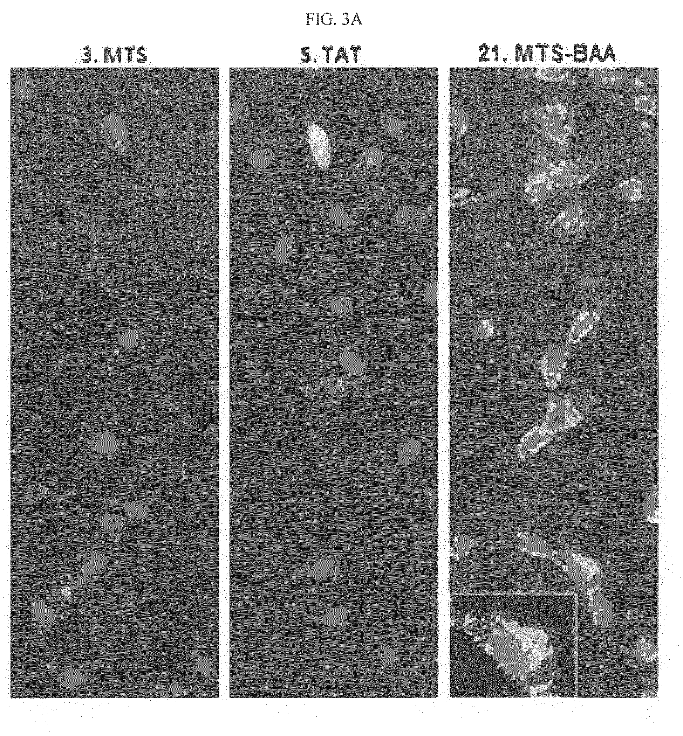

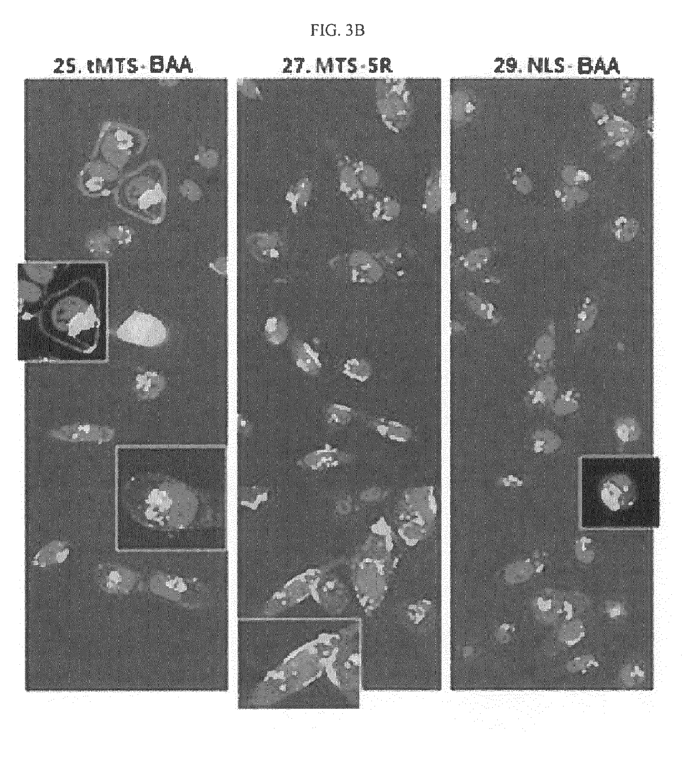

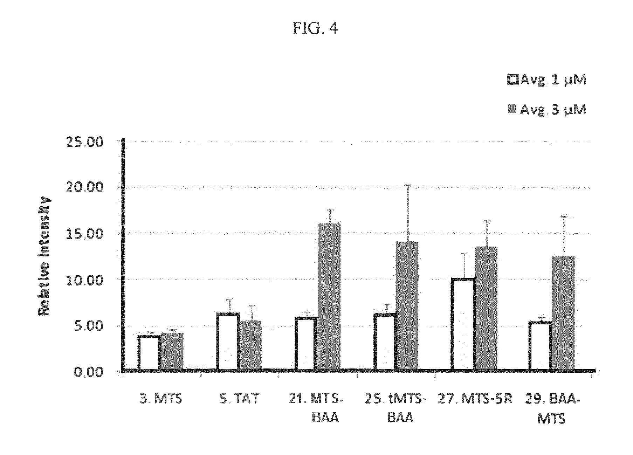

The obtained results are illustrated in FIGS. 3A, 3B, and 4. FIG. 4 is s graph illustrating numerical values obtained by quantifying fluorescent intensities shown in FIG.S 3A and 3B.

As shown in FIGS. 1-4, it can be concluded that MTS-BAA conjugates (prepared in Example 1.1) and various MTS-BAA family conjugates (prepared in Example 1.3) have more excellent cell membrane penetrability, compared to the conjugates (prepared in Example 1.2) including MTS or a basic peptide together with a cargo.

### Example 3. Test of Cell Uptake Patterns of the Cell Membrane Penetrating Conjugates

To examine cell uptake patterns of the MTS-BAA conjugates prepared in Example 1.1 in living MDA-MB-231 cells, imaging of the MTS-BAA conjugates in MDA-MB-231 cells was conducted in real time. MDA-MB-231 cells (10,000cells/well) were cultured in 8 well imaging chamber for 20 hours, and then, the nuclei were stained with Hoechst33342 (R37605; Invitrogen, Inc) according to a manufacturer's manual (as cell membrane (lipid) staining dye, CellMARK deepred (C10046) was used). Thereafter, images were obtained from the cells at 7.5 minute intervals. The cells were treated with each of the MTS-BAA conjugates at the concentration of 3 µM, and imaging to the conjugate treated cells was conducted. The imaging was conducted with X630 (total imaging magnification) and 2.5-fold digital zooming (LSM710, Carl Zeiss), and the obtained images were analyzed using image J and plotted. The imaging and image analysis were conducted referring to the method described in Example 2.

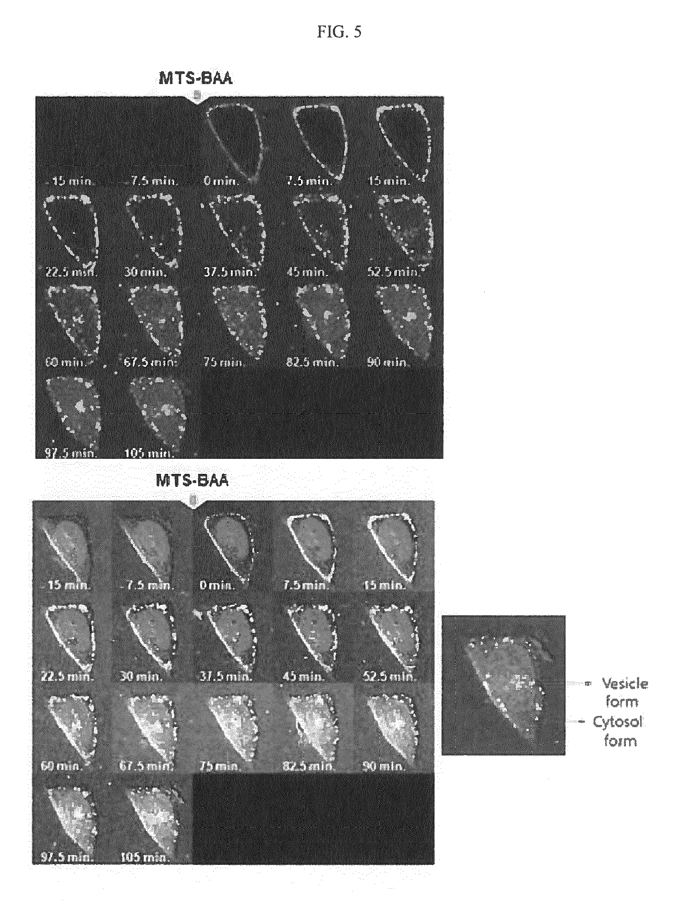

The obtained results are illustrated in FIG. 5. FIG. 5A contains fluorescent images obtained using FITC labeled peptide, and FIG. 5B contains the merged images. In FIG. 5, blue color indicates nuclei and green color indicates the MTS-BAA conjugates. As shown in FIG. 5, the MTS-BAA conjugates exhibit both patterns of a vesicle form and diffusion (cytosol) form, and they have not penetrated into nuclei.

### Example 4. Test of Cell Uptake of the Cell Membrane Penetrating Conjugates in Various Cells

Cell uptake of the MTS-BAA conjugate prepared in Example 1.1 was tested in various cell lines.

In this experimentation, breast cancer cell lines MDA-MB-231 (ATCC: HTB-26), MDA-MB-157 (ATCC: HTB-24), BT20 (ATCC: HTB-19), MCF7 (ATCC: HTB-22), HCC1806 (ATCC: CRL2335), and HCC1143 (ATCC: CRL2321), and colorectal cancer cell line HCT116 (ATCC: CCL247), were used. Each of MDA-MB-231 and MDA-MB-157 cell lines was cultured in 10% FBS/DMEM medium at the amount of 10,000cells, each of BT20 and MCF7 cell lines was cultured in 10% FBS/EMEM medium at the amount of 10,000cells, and each of HCC1806, HCC1143, and HCT116 cell lines was cultured in 10% FBS/RPMI1640 medium at the amount of 10,000cells, under the conditions of 5% CO₂ and 37°C (RMPI1640, DMEM: Invitrogen). Each of the cultured cell lines was treated with the MTS-BAA conjugate at the concentration of 1 µM or 2 µM for two hours. Images were obtained from the conjugate treated cells, analyzed using image J, and plotted. The imaging and image analysis were conducted referring to the method described in Example 2.

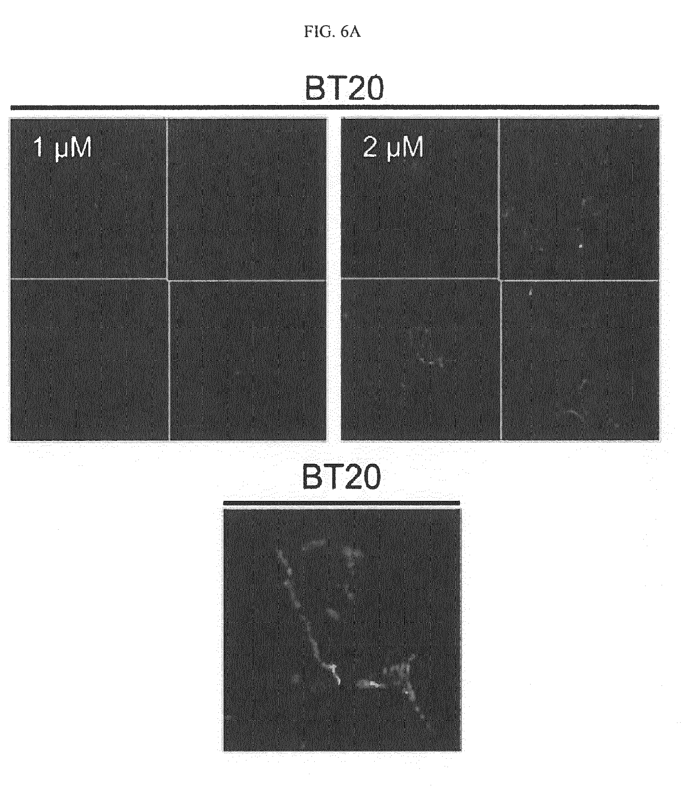

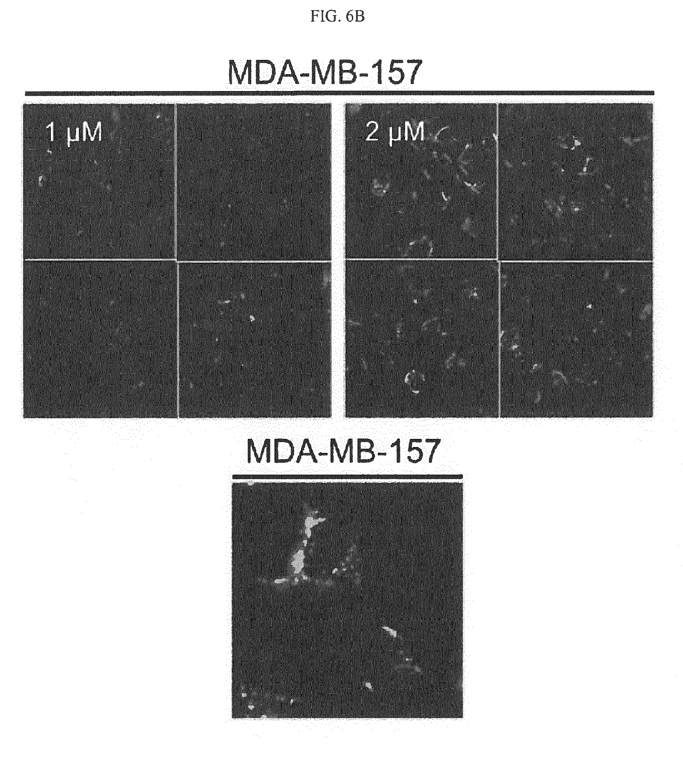

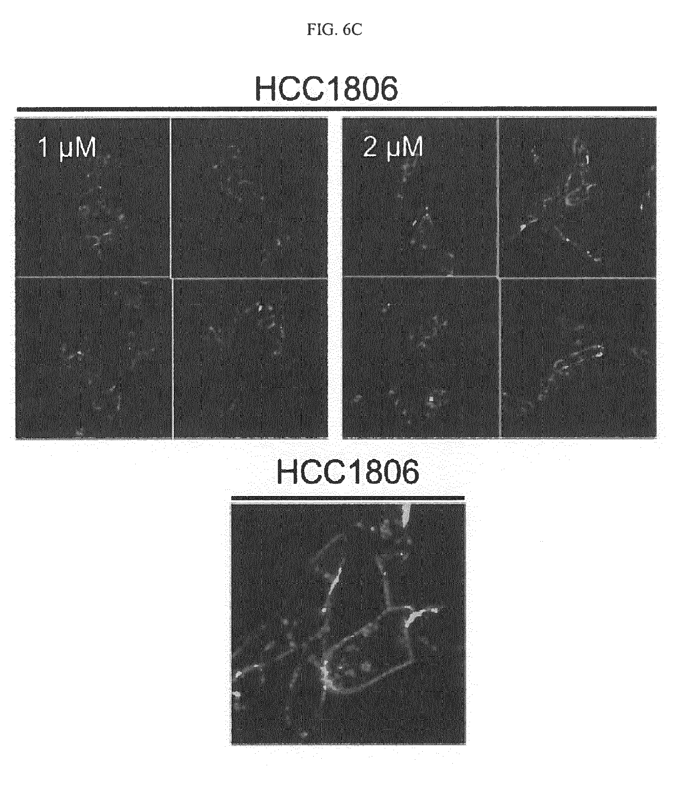

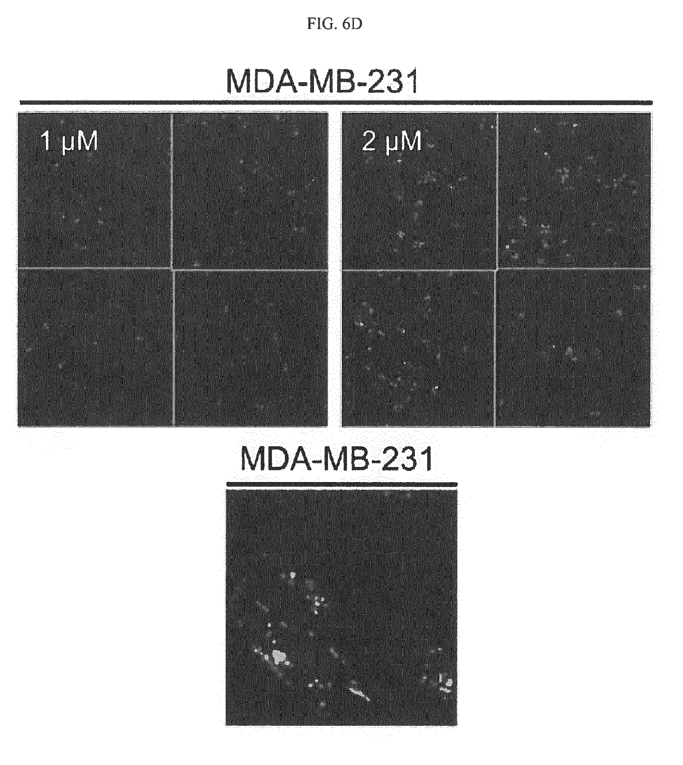

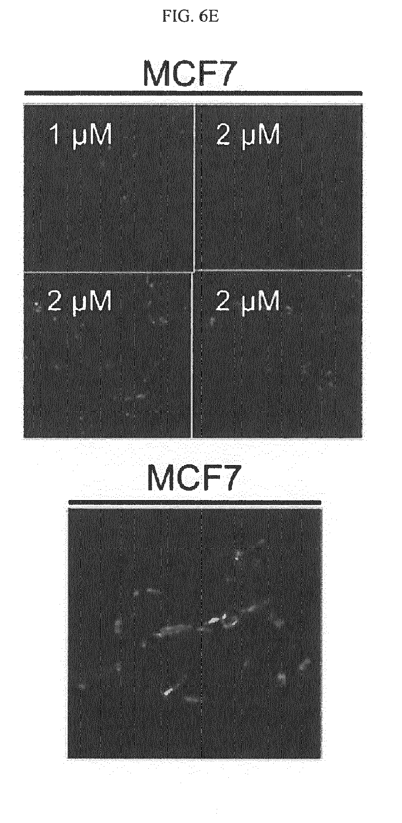

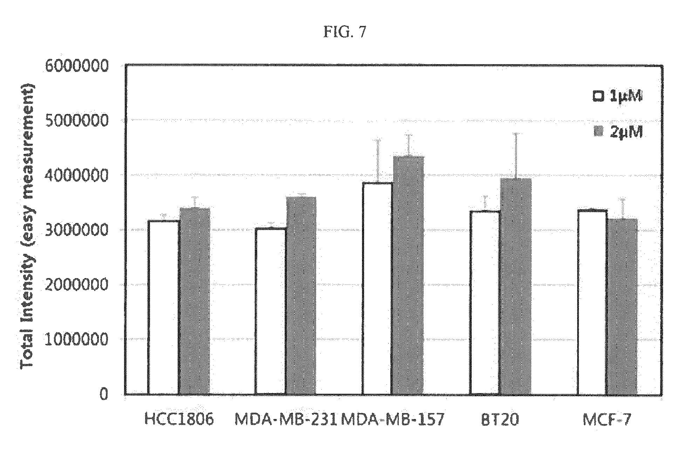

The obtained cell uptake results for breast cancer cell lines, MDA-MB-231, MDA-MB-157, BT20, MCF7, and HCC1806, are illustrated in FIGS 6A-6E (6A: BT20 cells, 6B: MDA-MB-157, 6C: HCC1806, 6D: MDA-MB-231, and 6E: MCF7) and 7. As shown in FIGS 6 and 7, in all the cell lines treated with the MTS-BAA conjugate, intracellular uptake of the conjugate was observed at the excellent level, indicating that the conjugate can be broadly applied to various cancer cells.

### Example 5. Test of Cell Uptake of the Various Cell Membrane Penetrating Conjugates

To reveal the role of proline in a hydrophobic peptide of SEQ ID NO: 1, the level of cell uptake of the cell membrane penetrating conjugates was measured depending on the presence or absence of proline. For this purpose, the level of cell uptake was tested using the MTS-BAA family conjugates (including truncated MTS-BAA conjugates) shown in Table 2 prepared in Example 1.3. Constructs 30, 31, and 32 of Table 2 included a hydrophobic peptide in which the last proline of SEQ ID NO: 1 was substituted with alanine.

MDA-MB-231 cells (ATCC, HTB-26; 10,000 cells) were cultured in 8 well imaging chamber(Ibidi) for 20 hours, and then, treated with each of the cell membrane penetrating conjugates of Table 2 in Example 1.3 at the concentration of 1 µM or 3 µM for 2 hours. Thereafter, the medium was exchanged with 10% (v/v) FBS containing RPMI1640 medium (Invitrogen). Imaging (total imaging magnification: X 630) was conducted to obtain images, and then, the obtained images was analyzed using image J (JAVA) and plotted. The imaging and image analysis were conducted referring to the method described in Example 2.

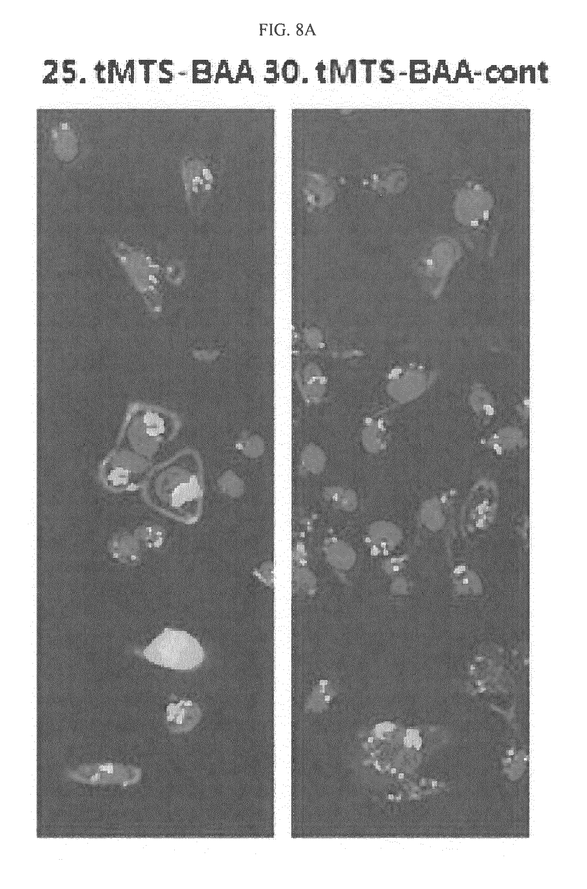

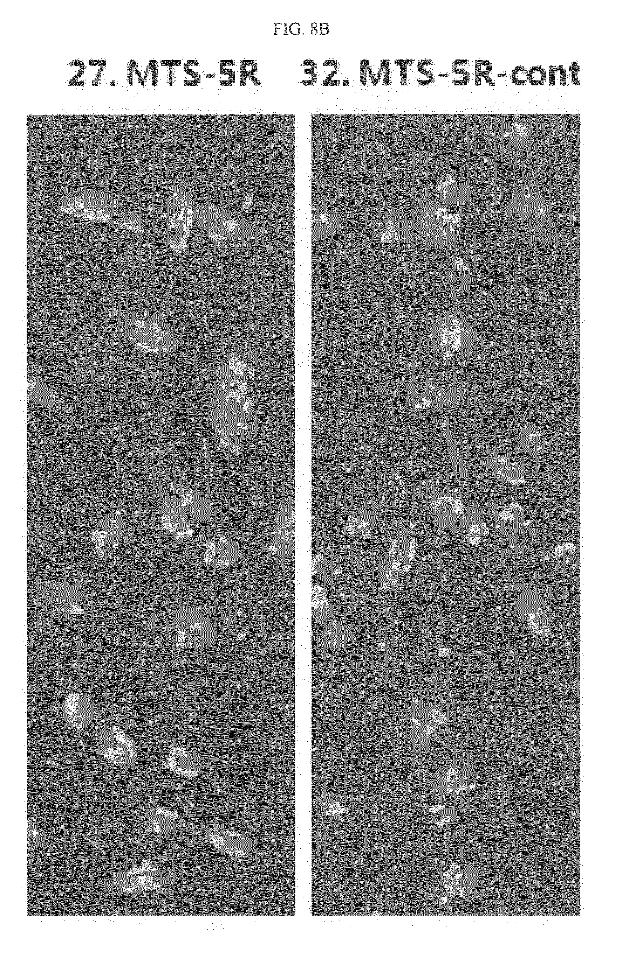

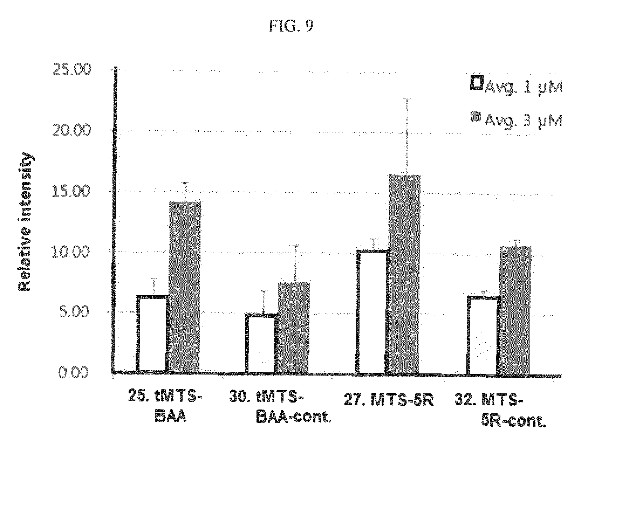

The obtained results are illustrated in FIGS. 8A, 8B, and 9. As shown in FIGS. 8A, 8B, and 9, when the conjugate (indicated as 'cont') includes a hydrophobic peptide that includes substitution of proline with alanine in the amino acid sequence of SEQ ID NO: 1 or a fragment thereof, the cell uptake level of the conjugate ('cont') is considerably decreased. The results indicate that proline in the amino acid sequence of SEQ ID NO: 1 plays an important role in cell membrane penetration.

### Example 6. Real-Time Observation of Cell Uptake of the Cell Membrane Penetrating Conjugate

The cell uptake level of the MTS-BAA conjugate prepared in Example 1.1 was examined for MCF7 breast cancer cell line. MCF7 breast cancer cells (ATCC, HTB-22; 10,000cells) were cultured in 8 well imaging chamber for 20 hours, and then, treated with the MTS-BAA conjugate prepared in Example 1.1 at the concentration of 2 µM for 1 hour. Imaging was conducted sequentially from 10 minutes before to 1 hour after the treatment of MTS-BAA conjugate, to obtain images, and the obtained images were analyzed using image J and plotted. The imaging and image analysis were conducted referring to the method described in Example 2.

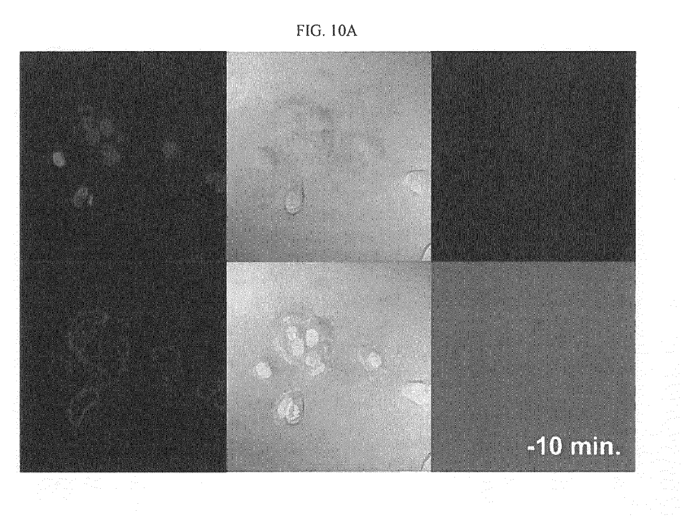

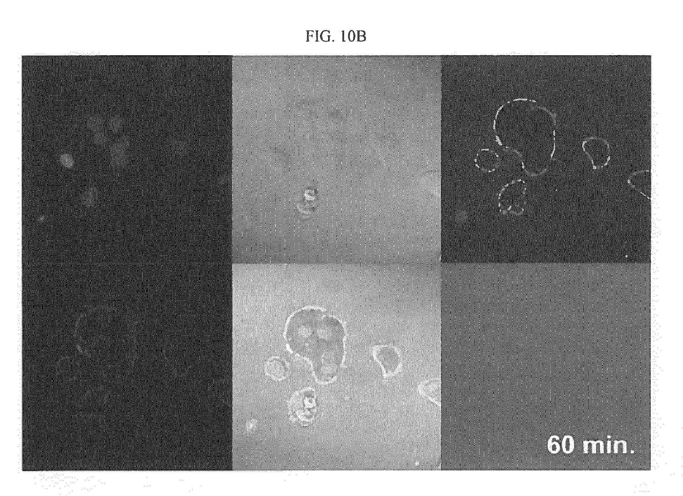

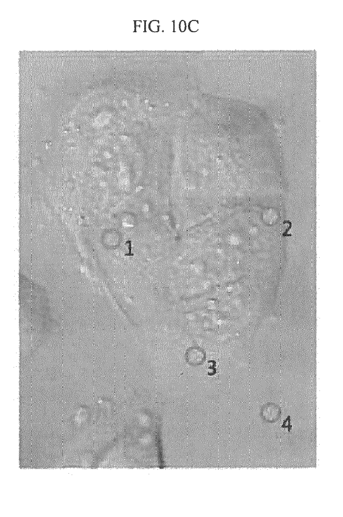

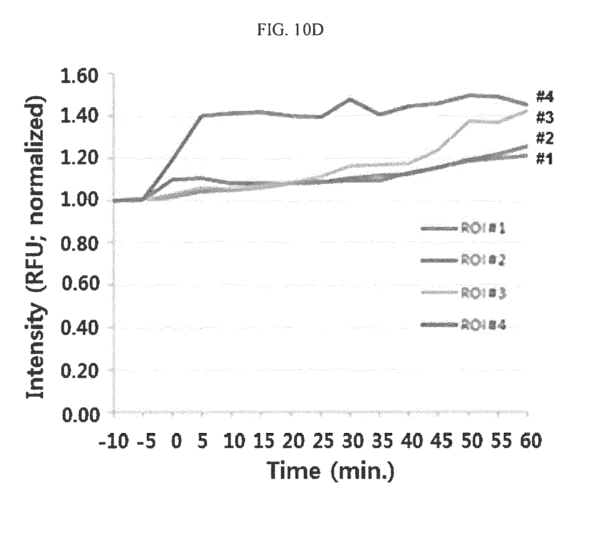

The obtained results are illustrated in FIGS. 10A-10D. The graph of FIG. 10C shows fluorescent intensities at the locations indicated in the image of FIG. 10C. FIGS. 10A-10D show that the MTS-BAA conjugate is detected at a high concentration at location R01#4 which is outside of the cells; and at locations of inside of cells, ROI#1, ROI#2, and ROI#3, the fluorescent intensities becomes gradually increased as the MTS-BAA conjugate is transferred into the cells. The results indicate that the MTS-BAA conjugate is delivered to the inside of the cells over time.

### Example 7: Anticancer Effect of Cell Membrane Penetrating Conjugate Including Anticancer Protein

To examine an anticancer effect of a cell membrane penetrating conjugate including an anticancer protein, MTS-BAA-p16M7 conjugate and TAT-p16M7 (for comparison) were prepared.

D(EGFR)-TEV-MTS-BAA-p16M7 [DARPin(EGFR)-TEV-(SEQ ID NO: 1)-(SEQ ID NO: 2)-(SEQ ID NO: 3)-p16M7] coding gene (synthesized by ANYGEN Co. Ltd. and PEPTRON Inc.) was subcloned into pET21b vector (Novagen) using NdeI/XhoI restriction enzyme, to produce pET21b:TAT-p16M7 plasmid and pET21b:D(EGFR)-TEV-MTS-NLS-p16M7 plasmid:
DARPin(EGFR) (SEQ ID NO: 19) 
DLGKKLLEAARAGQDDEVRILMANGADVNADDTWGWTPLHLAAYQGHLEIV
EVLLKNGADVNAYDYIGWTPLHLAADGHLEIVEVLLKNGADVNASDYIGDTPLHLAAH
NGHLEIVEVLLKHGADVNAQDKFGKTAFDISIDNGNEDLAEILQ
TEV(TEV cleavage site) (SEQ ID NO: 18)
   ENLYFQGS
p16M7 (a p16 variant with improved efficacy: SEQ ID NO: 16) 
MEPAAGSSMEPSADKLATAAARGRVEEVRALLEAGADPNAPNSYGRRPIQVM
MMGSARVAELLLKHGAEPNSADPATSTRPVHDAAREGFLDTLVVLHRAGARLDARDA
WGRTPVDLAEELGHRDVARYLRAAAGGTRGSNHARIDAAEGPSDIPD
TAT-p16M7 (SEQ ID NO: 20-SEQ ID NO: 16) was synthesized by ANYGEN Co. Ltd. and PEPTRON Inc.

As a competent cell, BL21(DE3)CodonPlus-RIPL (Stratagene) was used.

1 µL of each of the prepared plasmids was transfected into *E. coli* (Stratagene, 70ul). 2 mL of the obtained *E. coli* culture was added to 1L of LB medium (Sigma; Tryptone (pancreatic digest of casein) 10 g/L, Yeast extract 5 g/L, and NaCl 5 g/L), and cultured O/N(overnight) at 37°C. When the optical density at 600 nanometers (OD600) value of the culture reached about ∼0.8, the culture was treated with 1mM IPTG (Isopropyl-beta-D-thio-galactoside), and further cultured at18 for 15 hours.

1 L of the culture was collected, and centrifuged at 4000rpm at 4°C for 10 minutes. The supernatant was removed, and the precipitated cells were resuspended with 50 mL of lysis buffer (20mM Tris-HCl pH7.4, 200mM NaCl, 1mM PMSF). The cell resuspension was subjected to sonication (pulse 1s/1s, 70% amplitude, 1min, 4 times total), to lyse the cells. The lysed cells was centrifuged at 18000rpm at 4°C for 50 minutes, and the supernatant was collected and subjected to His-affinity chromatography using AKTA FPLC system (GE healthcare). Columns and buffers used for the chromatography are as follows:
Column: HisTrap crude FF, 5mL size, GE healthcare
Buffer: A=20mM Tris-HCl pH7.4, 200mM NaCl, B=20mM Tris-HCl pH7.4, 200mM NaCl, 500mM imidazole

The proteins adsorbed onto the column were eluted by linear gradient (from 0 to 500mM imidazole, 20CV length). The eluate was treated with TEV enzyme to remove N-terminal part of the conjugate (at 18°C and for 18 hours), and then, subjected to second His-affinity chromatography to collect non-binding fractions. Columns and buffers used for the chromatography are as follows:
Column: HisGraviTrap, 1mL size, GE healthcare
Buffer: 20mM Tris-HCl pH7.4, 200mM NaCl

The collected fractions were subjected to buffer exchange processes, and the medium was finally exchanged to RPMI, and the resultant was frozen and kept at -80°C. Through such processes, the MTS-BAA-p16 and Tat-p16 conjugates were purified, and the finally purified conjugates were condensed to the concentration to be required for the experiment using YM-10 (Millipore). Columns and buffers used for the chromatography are as follows:
Column: PD-10, GE healthcare
Buffer: RPMI (Gibco)

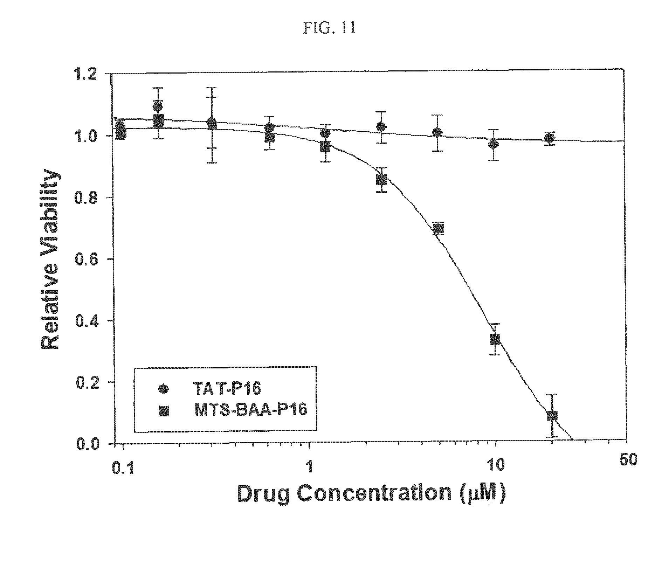

MDA-MB-231 cells were incubated at the amount of about 5,000-8,000 cells/well in 96 well plate for 24 hours, and then, treated with each of the obtained conjugates MTS-BAA-p16M7 and Tat-p16M7 at various concentrations (refer to FIG. 11). 24 hours or 72 hours after, the viability of the cells was measured by reading the fluorescent intensity of viable cells using titer® proliferation assay kit (Promega) according to manufacturer's manual. The results were normalized based on the results from a control (non-treated) group, and plotted (the value of the control group: "1"). The experiment was conducted in triplicate, and the error was treated using a standard deviation.

The obtained results (results obtained at 72 hours after the conjugate treatment) are illustrated in FIG. 11. As shown in FIG. 11, Tat-p16M7 conjugate exhibits nearly no inhibitory effect on cancer cell proliferation regardless of the treatment concentration, whereas MTS-BAA-p16M7 conjugate exhibits considerable inhibitory effect on cancer cell proliferation in a concentration dependent manner. Such inhibitory effect on cancer cell proliferation indicates that MTS-BAA-p16M7 conjugate according to one embodiment of the present disclosure has high intracellular delivery (cell membrane penetration) efficacy, thereby exhibiting excellent cancer cell proliferation inhibition effect (anticancer effect). However, TAT peptide, which is one of commonly used cell membrane penetrating peptides, exhibits very low efficacy of intracellular delivery of a protein of 16KDa.

It should be understood that the exemplary embodiments described herein should be considered in a descriptive sense only and not for purposes of limitation. Descriptions of features or aspects within each embodiment should typically be considered as available for other similar features or aspects in other embodiments.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A fusion peptide comprising a hydrophobic peptide and a basic peptide which are linked to each other, wherein
the hydrophobic peptide consists of 5 to 40 amino acids of which 70% or more are hydrophobic amino acids, wherein each hydrophobic amino acid is independently selected from the group consisting of glycine, alanine, valine, leucine, isoleucine, methionine, proline, tryptophan, and phenylalanine, and
the basic peptide consists of 2 to 6 basic amino acids, wherein each basic amino acid is independently selected from the group consisting of lysine, arginine, and histidine.

2. The fusion peptide of claim 1, wherein the hydrophobic peptide comprises SEQ ID NO: 1; a 7-16 amino acid fragment of SEQ ID NO: 1; SEQ ID NO: 17; SEQ ID NO: 26; or a combination thereof.

3. The fusion peptide of claim 1 or 2, wherein the basic peptide consists of 2 to 6 amino acids which are independently selected from the group consisting of lysine and arginine.

4. The fusion peptide of any one of claims 1 to 3, wherein the basic peptide comprises at least one selected from the group consisting of the peptides of SEQ ID NO: 2, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13.

5. The fusion peptide of any one of claims 1 to 4, wherein the basic peptide is linked to the C-terminus of the hydrophobic peptide.

6. A pharmaceutical composition comprising the fusion peptide of any one of claims 1 to 5 and a pharmaceutically acceptable carrier.

7. A cell membrane penetrating conjugate comprising the fusion peptide of any one of claims 1 to 5 and a bioactive substance or contrast material linked to the fusion peptide.

8. The cell membrane penetrating conjugate of claim 7, further comprising a second basic peptide at the N-terminus or C-terminus of the conjugate, wherein the second basic peptide consisting of 2 to 6 basic amino acids wherein each basic amino acid is independently selected from the group consisting of lysine, arginine, and histidine.

9. The cell membrane penetrating conjugate of claim 7 or claim 8 for the use in delivering the bioactive substance or the contrast material into a subject.

10. The cell membrane penetrating conjugate of claim 7 or claim 8 for the use in improving cell membrane penetrability of the bioactive substance or the contrast material.

11. A method of preparing the fusion peptide of any one of claim 1 to 5, comprising linking a basic peptide to the N-terminus, the C-terminus, or both termini of a hydrophobic peptide.

12. A pharmaceutical composition for delivering a bioactive substance or contrast material or improving cell membrane penetrability of a bioactive substance or contrast material, comprising the conjugate of claim 7 or claim 8 and a pharmaceutically acceptable carrier.


<110>    SAMSUNG ELECTRONICS CO., LTD.

<120>    Fusion peptide and use thereof for cell membrane penetrating

<130>    EP97082FZSEpau

<140>    not yet assigned
<141>    herewith

<150>    KR 10-2013-0129482
<151>    2013-10-29

<160>    26

<170>    KopatentIn 1.71

<210>    1
<211>    16
<212>    PRT
<213>    Artificial Sequence

<220>
<223>    membrane-translocation sequence (MTS)


<400>    1
Ala Ala Val Ala Leu Leu Pro Ala Val Leu Leu Ala Leu Leu Ala Pro
  1               5                  10                  15


<210>    2
<211>    5
<212>    PRT
<213>    Artificial Sequence

<220>
<223>    Unit of basic amino acid (BAA)


<400>    2
Lys Lys Lys Arg Lys
  1               5


<210>    3
<211>    12
<212>    PRT
<213>    Artificial Sequence

<220>
<223>    pMI-beta


<400>    3
Thr Ala Trp Tyr Ala Asn Phe Glu Lys Leu Leu Arg
  1               5                  10


<210>    4
<211>    7
<212>    PRT
<213>    Artificial Sequence

<220>
<223>    MTS fragment

<400>    4
Ala Ala Val Ala Leu Leu Pro
1               5

<210>    5
<211>    9
<212>    PRT
<213>    Artificial Sequence

<220>
<223>    MTS fragment

<400>    5
Ala Val Leu Leu Ala Leu Leu Ala Pro
1               5

<210>    6
<211>    4
<212>    PRT
<213>    Artificial Sequence

<220>
<223>    Unit of basic amino acid

<400>    6
Lys Lys Lys Arg
1

<210>    7
<211>    4
<212>    PRT
<213>    Artificial Sequence

<220>
<223>    Unit of basic amino acid

<400>    7
Arg Lys Arg Lys
1

<210>    8
<211>    6
<212>    PRT
<213>    Artificial Sequence

<220>
<223>    Unit of basic amino acid

<400>    8
Arg Lys Arg Lys Arg Lys
1               5

<210>    9
<211>    5
<212>    PRT
<213>    Artificial Sequence

<220>
<223>    Unit of basic amino acid


<400>    9
Lys Lys Lys Lys Lys
1               5


<210>    10
<211>    6
<212>    PRT
<213>    Artificial Sequence

<220>
<223>    Unit of basic amino acid


<400>    10
Lys Lys Lys Lys Lys Arg
1               5


<210>    11
<211>    6
<212>    PRT
<213>    Artificial Sequence

<220>
<223>    Unit of basic amino acid


<400>    11
Lys Lys Lys Arg Lys Arg
1               5


<210>    12
<211>    5
<212>    PRT
<213>    Artificial Sequence

<220>
<223>    Unit of basic amino acid


<400>    12
Arg Arg Arg Arg Arg
1               5


<210>    13
<211>    6
<212>    PRT
<213>    Artificial Sequence

<220>
<223>    Unit of basic amino acid


<400>    13
Arg Arg Arg Arg Arg Arg
1               5


<210>    14
<211>    12
<212>    PRT
<213>    Artificial Sequence

<220>
<223>    pMI-alpha


<400>    14
Thr Asn Trp Tyr Ala Asn Leu Glu Lys Leu Leu Arg
1               5                   10


<210>    15
<211>    15
<212>    PRT
<213>    Artificial Sequence

<220>
<223>    p53 fragment


<400>    15
Ser Gln Glu Thr Phe Ser Asp Leu Trp Lys Leu Leu Pro Glu Asn
1               5                   10                  15


<210>    16
<211>    156
<212>    PRT
<213>    Artificial Sequence

<220>
<223>    p16 variant (p16M7)


<400>    16
Met Glu Pro Ala Ala Gly Ser Ser Met Glu Pro Ser Ala Asp Lys Leu
1               5                   10                  15

Ala Thr Ala Ala Ala Arg Gly Arg Val Glu Glu Val Arg Ala Leu Leu
            20                  25                  30

Glu Ala Gly Ala Asp Pro Asn Ala Pro Asn Ser Tyr Gly Arg Arg Pro
        35                  40                  45

Ile Gln Val Met Met Met Gly Ser Ala Arg Val Ala Glu Leu Leu Leu
    50                  55                  60

Lys His Gly Ala Glu Pro Asn Ser Ala Asp Pro Ala Thr Ser Thr Arg
65                  70                  75                  80

Pro Val His Asp Ala Ala Arg Glu Gly Phe Leu Asp Thr Leu Val Val

85                  90                  95

Leu His Arg Ala Gly Ala Arg Leu Asp Ala Arg Asp Ala Trp Gly Arg
            100                 105                 110

Thr Pro Val Asp Leu Ala Glu Glu Leu Gly His Arg Asp Val Ala Arg
        115                 120                 125

Tyr Leu Arg Ala Ala Ala Gly Gly Thr Arg Gly Ser Asn His Ala Arg
    130                 135                 140

Ile Asp Ala Ala Glu Gly Pro Ser Asp Ile Pro Asp
145                 150                 155


<210>   17
<211>   9
<212>   PRT
<213>   Artificial Sequence

<220>
<223>   tMTS


<400>   17
Ala Val Leu Leu Ala Leu Leu Ala Ala
1               5


<210>   18
<211>   8
<212>   PRT
<213>   Artificial Sequence

<220>
<223>   TEV cleavage site


<400>   18
Glu Asn Leu Tyr Phe Gln Gly Ser
1               5


<210>   19
<211>   153
<212>   PRT
<213>   Artificial Sequence

<220>
<223>   DARPin(EGFR)


<400>   19
Asp Leu Gly Lys Lys Leu Leu Glu Ala Ala Arg Ala Gly Gln Asp Asp
1               5                   10                  15

Glu Val Arg Ile Leu Met Ala Asn Gly Ala Asp Val Asn Ala Asp Asp
            20                  25                  30

Thr Trp Gly Trp Thr Pro Leu His Leu Ala Ala Tyr Gln Gly His Leu
        35                  40                  45

Glu Ile Val Glu Val Leu Leu Lys Asn Gly Ala Asp Val Asn Ala Tyr

50                  55                  60

Asp Tyr Ile Gly Trp Thr Pro Leu His Leu Ala Ala Asp Gly His Leu
65                  70                  75                  80

Glu Ile Val Glu Val Leu Leu Lys Asn Gly Ala Asp Val Asn Ala Ser
                85                  90                  95

Asp Tyr Ile Gly Asp Thr Pro Leu His Leu Ala Ala His Asn Gly His
            100                 105                 110

Leu Glu Ile Val Glu Val Leu Leu Lys His Gly Ala Asp Val Asn Ala
        115                 120                 125

Gln Asp Lys Phe Gly Lys Thr Ala Phe Asp Ile Ser Ile Asp Asn Gly
    130                 135                 140

Asn Glu Asp Leu Ala Glu Ile Leu Gln
145                 150


<210>   20
<211>   9
<212>   PRT
<213>   Artificial Sequence

<220>
<223>   TAT peptide


<400>   20
Arg Lys Lys Arg Arg Gln Arg Arg Arg
1               5


<210>   21
<211>   9
<212>   PRT
<213>   Artificial Sequence

<220>
<223>   MTD103


<400>   21
Leu Ala Leu Pro Val Leu Leu Leu Ala
1               5


<210>   22
<211>   21
<212>   PRT
<213>   Artificial Sequence

<220>
<223>   TP10


<400>   22
Ala Gly Tyr Leu Leu Gly Lys Ile Asn Leu Lys Ala Leu Ala Ala Leu
1               5                   10                  15

Ala Lys Lys Ile Leu

            20

<210>    23
<211>    16
<212>    PRT
<213>    Artificial Sequence

<220>
<223>    Penetratin

<400>    23
Arg Gln Ile Lys Ile Trp Phe Gln Asn Arg Arg Met Lys Trp Lys Lys
1               5                   10                  15

<210>    24
<211>    9
<212>    PRT
<213>    Artificial Sequence

<220>
<223>    R9

<400>    24
Arg Arg Arg Arg Arg Arg Arg Arg Arg
1               5

<210>    25
<211>    18
<212>    PRT
<213>    Artificial Sequence

<220>
<223>    MAP

<400>    25
Lys Leu Ala Leu Lys Leu Ala Leu Lys Ala Leu Lys Ala Ala Leu Lys
1               5                   10                  15

Leu Ala

<210>    26
<211>    16
<212>    PRT
<213>    Artificial Sequence

<220>
<223>    MTS variant

<400>    26
Ala Ala Val Ala Leu Leu Pro Ala Val Leu Leu Ala Leu Leu Ala Ala
1               5                   10                  15